# EUROPEAN PATENT APPLICATION

(11) **EP 2 860 177 A2**
(43) Date of publication of application: **15.04.2015**
(21) Application number: 14184874.7
(22) Date of filing: 16.09.2014
(51) Int. Cl.: C07D 309/12, C07C 315/04, C07C 317/22

(54) **Synthesis of functionalized arenes**

(30) Priority: 20.09.2013 EP 13185397; 22.10.2013 EP 13189613
(71) Applicant: Bayer Intellectual Property GmbH, 40789 Monheim am Rhein (DE)
(72) Inventor: Mais, Franz-Josef, 40591 Düsseldorf (DE)
(74) Representative: BIP Patents

(57) **Abstract**

The present invention relates to methods of preparing (cyclo)alkoxy-((cyclo)alkylsulfanyl)benzenes, (cyclo)alkoxy-((cyclo)alkylsulfinyl)benzenes and (cyclo)alkoxy-((cyclo)alkylsulfonyl)benzenes of general formula (I):

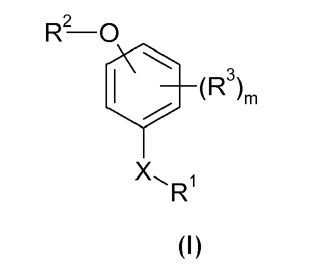

as well as to intermediate compounds useful in the preparation of said compounds.

## Description

The present invention relates to methods of preparing alkoxy-(alkylsulfanyl)benzenes, alkoxy-(alkylsulfinyl)benzenes, and alkoxy-(alkylsulfonyl)benzenes as described and defined herein, as well as to intermediate compounds useful in the preparation of said compounds.

### BACKGROUND OF THE INVENTION

Alkoxy-(alkylsulfanyl)benzenes, alkoxy-(alkylsulfinyl)benzenes and alkoxy-(alkylsulfonyl)benzenes of general formula (I) are important intermediates for the preparation of pharmaceuticals (see e.g. WO2013/087579A1, WO2012/143329A1, WO2011/064328A1, WO2009/086138A1, WO2005/073244A1): 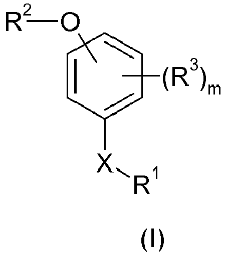 in which :
- X: represents S, S(=O) or S(=O)₂;
- R¹: represents a group selected from: C₁-C₆-alkyl-, C₃-C₆-cycloalkyl-;
- R²: represents a group selected from: C₁-C₆-alkyl-, halo-C₁-C₆-alkyl, C₃-C₆-cycloalkyl-;
- R³: represents a halogen atom or a group selected from: C₁-C₆-alkyl-, C₃-C₆-cycloalkyl-;
- m: represents an integer of 0, 1, 2, 3 or 4.

In WO2013/087579A1 a method for the preparation of 1-bromo-2-methoxy-4-(methylsulfanyl)benzene is described: sodium methanethiolate is added to a stirred solution of 1-bromo-4-fluoro-2-methoxybenzene in DMF. The mixture is stirred at room temperature for 30 minutes and at 85 °C for 2 h. Water is added and the mixture is extracted with ethyl acetate.

It turned out that the methoxy group is hydrolysed to a significant extent - resulting in the formation of the respective phenol:

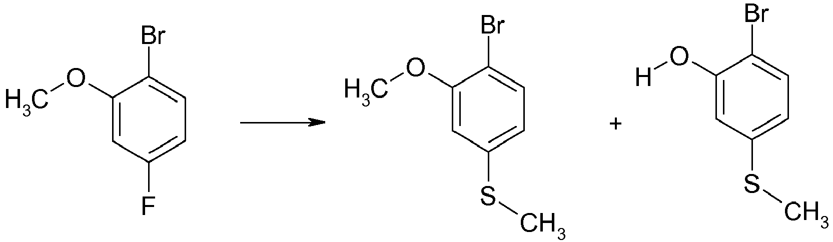

The removal of the phenol from the desired 1-bromo-2-methoxy-4-(methylsulfanyl)benzene turned out to be laborious; the final yield of the desired product is low.

### SUMMARY of the INVENTION

The present invention is related to a method for preparing a compound of general formula (I): 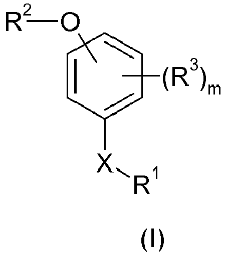 in which :
- X: represents S, S(=O) or S(=O)₂;
- R¹: represents a group selected from: C₁-C₆-alkyl-, C₃-C₆-cycloalkyl-;
- R²: represents a group selected from: C₁-C₆-alkyl-, halo-C₁-C₆-alkyl-, C₃-C₆-cycloalkyl-;
- R³: represents chloro, bromo or iodo, or a group selected from: C₁-C₆-alkyl-, C₃-C₆-cycloalkyl-; and
- m: represents an integer of 0, 1, 2, 3 or 4;
the method being characterized in that an intermediate compound of general formula (III): 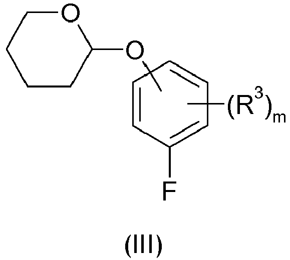 in which m and R³ are as defined for the compounds of general formula (I), *supra;*
is allowed to react with an alkanethiolat of general formula (IV):

R¹-S⁻X⁺ (IV)

in which R¹ is as defined for the compounds of general formula (I), *supra,* and X⁺ is a counter ion to the negatively charged sulphur ion, preferably Na⁺;
thus providing a compound of formula (V):

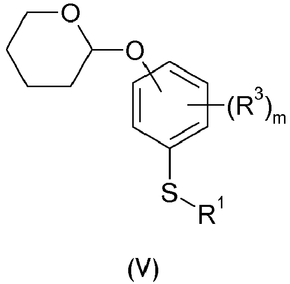

The present invention further relates to intermediate compounds useful in the preparation of the compounds of formula (I), *supra,* in particular to intermediate compounds of formula (III) and intermediate compounds of formula (V), *supra,* as well as of compounds of formulae (VI), (VII), (VIII), and (X) in which m, R¹ and R³ are as defined for the compounds of general formula (I):

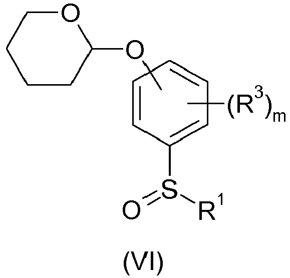

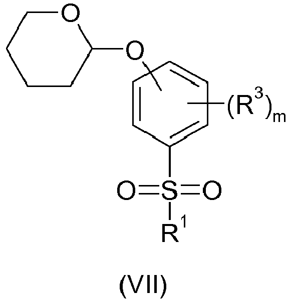

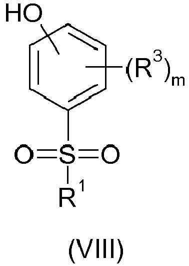

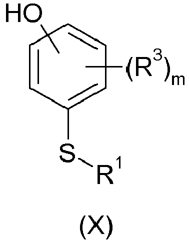

### DETAILED DESCRIPTION of the INVENTION

The terms as mentioned in the present text have preferably the following meanings :
The term "halogen atom" or "halo-" is to be understood as meaning a fluorine, chlorine, bromine or iodine atom.

The term "C₁-C₆-alkyl" is to be understood as preferably meaning a linear or branched, saturated, monovalent hydrocarbon group having 1, 2, 3, 4, 5 or 6 carbon atoms, *e.g.* a methyl, ethyl, propyl, butyl, pentyl, hexyl, iso-propyl, iso-butyl, sec-butyl, tert-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl group, or an isomer thereof. Particularly, said group has 1, 2, 3 or 4 carbon atoms ("C₁-C₄-alkyl"), e.g. a methyl, ethyl, propyl, butyl, iso-propyl, iso-butyl, sec-butyl, tert-butyl group, more particularly 1, 2 or 3 carbon atoms ("C₁-C₃-alkyl"), *e.g.* a methyl, ethyl, n-propyl- or iso-propyl group.

The term "halo-C₁-C₆-alkyl" is to be understood as preferably meaning a linear or branched, saturated, monovalent hydrocarbon group in which the term "C₁-C₆-alkyl" is defined *supra,* and in which one or more hydrogen atoms is replaced by a halogen atom, in identically or differently, *i.e.* one halogen atom being independent from another. Particularly, said halogen atom is F. Said halo-C₁-C₆-alkyl group is, for example, -CF₃, -CHF₂, -CH₂F, -CF₂CF₃, or -CH₂CF₃.

The term "C₃-C₆-cycloalkyl" is to be understood as preferably meaning a saturated, monovalent, monocyclic hydrocarbon ring which contains 3, 4, 5 or 6 carbon atoms. Said C₃-C₆-cycloalkyl group is for example a cyclopropyl, cyclobutyl, cyclopentyl, or cyclohexyl ring.

The term "C₁-C₆", as used throughout this text, e.g. in the context of the definition of "C₁-C₆-alkyl", "C₁-C₆-haloalkyl", "C₁-C₆-alkoxy", or "C₁-C₆-haloalkoxy" is to be understood as meaning an alkyl group having a finite number of carbon atoms of 1 to 6, *i.e.* 1, 2, 3, 4, 5, or 6 carbon atoms. It is to be understood further that said term "C₁-C₆" is to be interpreted as any sub-range comprised therein, *e.g.* C₁-C₆, C₂-C₅, C₃-C₄, C₁-C₂, C₁-C₃, C₁-C₄, C₁-C₅, C₁-C₆; particularly C₁-C₂, C₁-C₃, C₁-C₄, C₁-C₅, C₁-C₆ ; more particularly C₁-C₄ ; in the case of "C₁-C₆-haloalkyl" or "C₁-C₆-haloalkoxy" even more particularly C₁-C₂.

Further, as used herein, the term "C₃-C₆", as used throughout this text, e.g. in the context of the definition of "C₃-C₆-cycloalkyl", is to be understood as meaning a cycloalkyl group having a finite number of carbon atoms of 3 to 6, *i.e.* 3, 4, 5 or 6 carbon atoms. It is to be understood further that said term "C₃-C₆" is to be interpreted as any sub-range comprised therein, e.g. C₃-C₆, C₄-C₅ , C₃-C₅ , C₃-C₄, C₄-C₆, C₅-C₆; particularly C₃-C₆.

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulphur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl ⁸²Br, ¹²³I, ¹²⁴I, ¹²⁹I and ¹³¹I, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

The present invention covers a method for the preparation of a compound of formula (I): 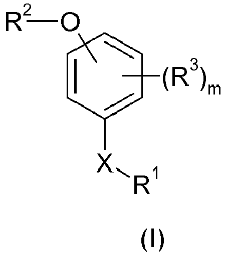 in which :
- X: represents S, S(=O) or S(=O)₂;
- R¹: represents a group selected from: C₁-C₆-alkyl-, C₃-C₆-cycloalkyl-;
- R²: represents a group selected from: C₁-C₆-alkyl-, halo-C₁-C₆-alkyl-, C₃-C₆-cycloalkyl-;
- R³: represents chloro, bromo or iodo, or a group selected from: C₁-C₆-alkyl-, C₃-C₆-cycloalkyl-; and
- m: represents an integer of 0, 1, 2, 3 or 4.

The method according to the invention comprises the step of converting a compound of general formula (II): 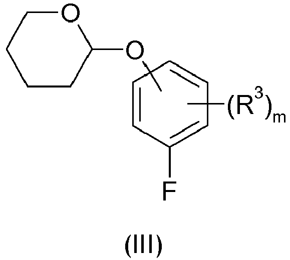 in which m and R³ are as defined for the compounds of general formula (I), *supra;* into a compound of general fomula (V): 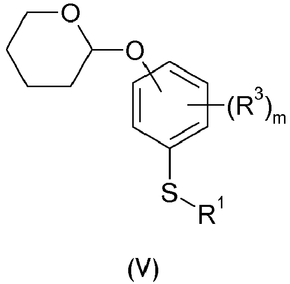 in which m, R¹ and R³ are as defined for the compounds of general formula (I), *supra.*

In a preferred embodiment, X represents S.

In another preferred embodiment, X represents S(=O).

In another preferred embodiment, X represents S(=O)₂.

In another preferred embodiment, R¹ represents a C₁-C₆-alkyl- group.

In another preferred embodiment, R¹ represents a C₃-C₆-cycloalkyl- group.

In another preferred embodiment, R¹ represents a C₁-C₃-alkyl- group.

In another preferred embodiment, R¹ represents a methyl or ethyl group.

In another preferred embodiment, R¹ represents a methyl group.

In another preferred embodiment, R² represents a C₁-C₆-alkyl- group.

In another preferred embodiment, R² represents a halo-C₁-C₆-alkyl- group.

In another preferred embodiment, R² represents a C₃-C₆-cycloalkyl- group.

In another preferred embodiment, R² represents a C₁-C₃-alkyl- group.

In another preferred embodiment, R² represents a methyl or ethyl group.

In another preferred embodiment, R² represents a methyl group.

In another preferred embodiment, R³ represents chloro, bromo or iodo.

In another preferred embodiment, R³ represents a group selected from: C₁-C₆-alkyl-, C₃-C₆-cycloalkyl-.

In another preferred embodiment, R³ represents bromo.

In another preferred embodiment, m represents an integer of 0, 1, or 2.

In another preferred embodiment, m represents an integer of 1.

In another preferred embodiment, the method comprises the following step:

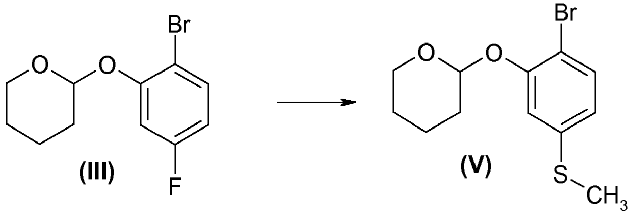

In accordance with a first aspect, the present invention covers a method for preparing a compound of general formula (Ia): 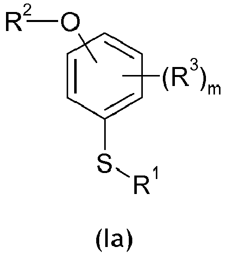 in which :
- R¹: represents a group selected from: C₁-C₆-alkyl-, C₃-C₆-cycloalkyl-;
- R²: represents a group selected from: C₁-C₆-alkyl-, halo-C₁-C₆-alkyl-, C₃-C₆-cycloalkyl-;
- R³: represents chloro, bromo or iodo, or a group selected from: C₁-C₆-alkyl-, C₃-C₆-cycloalkyl-; and
- m: represents an integer of 0, 1, 2, 3 or 4;
the method being characterized in that an intermediate compound of general formula (III): 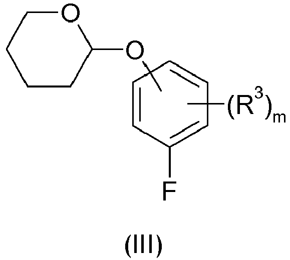 in which m and R³ are as defined for the compound of general formula (Ia), *supra;*
is allowed to react with an alkanethiolat of general formula (IV):

R¹-S⁻X⁺ (IV)

in which R¹ is as defined for the compound of general formula (Ia), *supra,* and X⁺ is a counter ion to the negatively charged sulphur ion, preferably Na⁺;
thus providing a compound of formula (V):

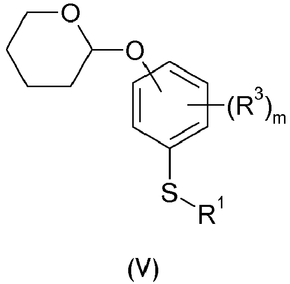

The whole process for the preparation of a compound of general formula (Ia) is depicted in the following scheme 1:

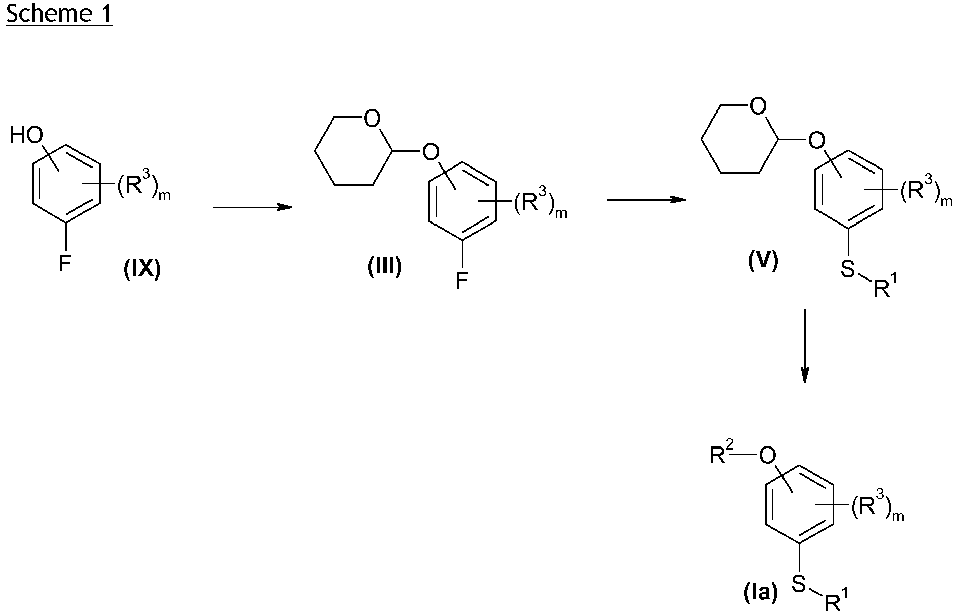

In accordance with a second aspect, the present invention covers a method for preparing a compound of general formula (Ib): 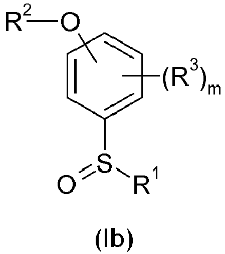 in which :
- R¹: represents a group selected from: C₁-C₆-alkyl-, C₃-C₆-cycloalkyl-;
- R²: represents a group selected from: C₁-C₆-alkyl-, halo-C₁-C₆-alky-l, C₃-C₆-cycloalkyl-;
- R³: represents chloro, bromo or iodo, or a group selected from: C₁-C₆-alkyl-, C₃-C₆-cycloalkyl-; and
- m: represents an integer of 0, 1, 2, 3 or 4;
the method being characterized in that an intermediate compound of general formula (III): 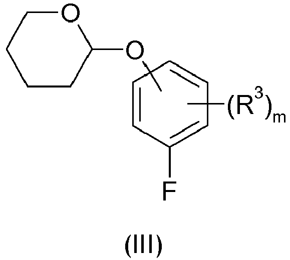 in which m and R³ are as defined for the compound of general formula (Ib), *supra;*
is allowed to react with an alkanethiolat of general formula (IV):

R¹-S⁻X⁺ (IV)

in which R¹ is as defined for the compound of general formula (Ib), *supra,* and X⁺ is a counter ion to the negatively charged sulphur ion, preferably Na⁺;
thus providing a compound of formula (V):

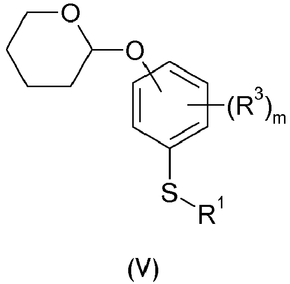

The whole process for the preparation of a compound of general formula (Ib) is depicted in the following scheme 2:

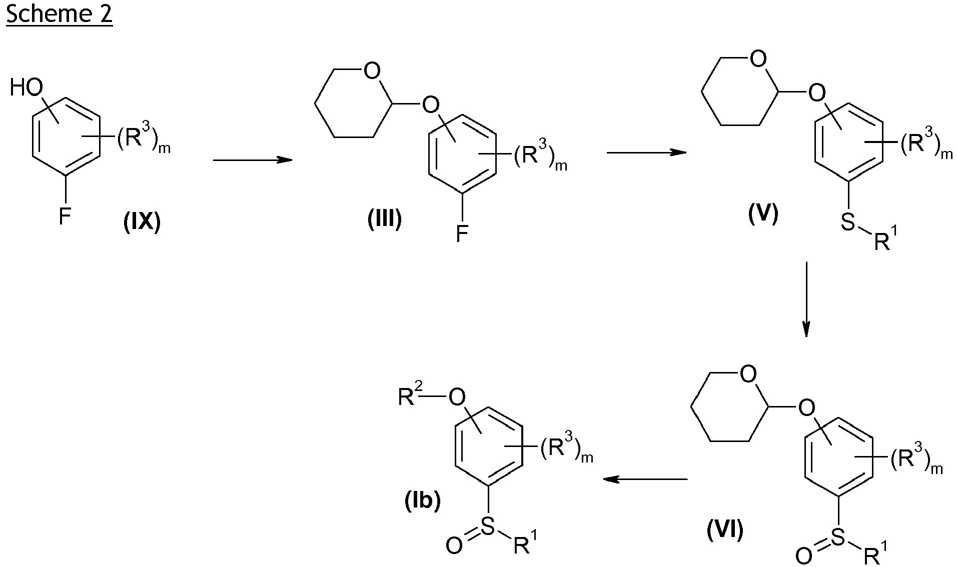

In accordance with a third aspect, the present invention covers a method for preparing a compound of general formula (Ic): 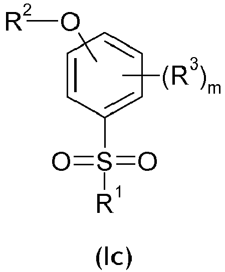 in which :
- R¹: represents a group selected from: C₁-C₆-alkyl-, C₃-C₆-cycloalkyl-;
- R²: represents a group selected from: C₁-C₆-alkyl-, halo-C₁-C₆-alkyl-, C₃-C₆-cycloalkyl-;
- R³: represents chloro, bromo or iodo, or a group selected from: C₁-C₆-alkyl-, C₃-C₆-cycloalkyl-; and
- m: represents an integer of 0, 1, 2, 3 or 4;
the method being characterized in that an intermediate compound of general formula (III): 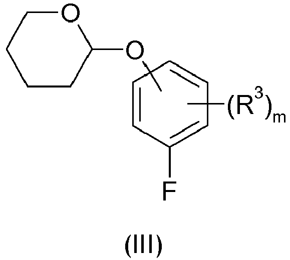 in which m and R³ are as defined for the compound of general formula (Ic), *supra;*
is allowed to react with an alkanethiolat of general formula (IV):

R¹-S⁻X⁺ (IV)

in which R¹ is as defined for the compound of general formula (Ic), *supra,* and X⁺ is a counter ion to the negatively charged sulphur ion, preferably Na⁺;
thus providing a compound of formula (V):

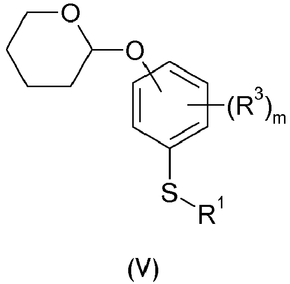

The whole process for the preparation of a compound of general formula (Ic) is depicted in the following scheme 3:

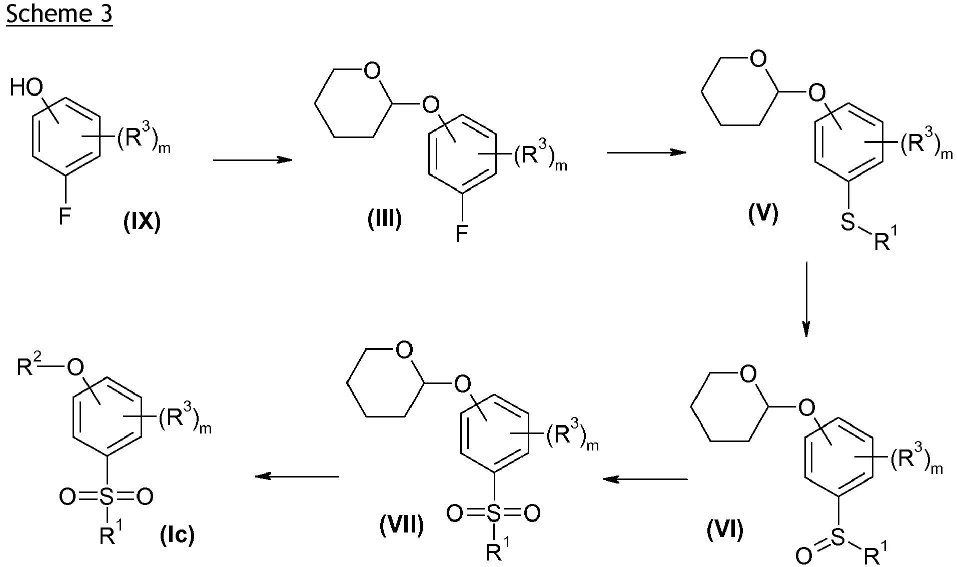

In accordance with another aspect, the present invention covers methods of preparing compounds of general formula (I), said methods comprising the steps as described in the Experimental Section herein.

In accordance with another aspect, the present invention covers intermediate compounds useful in the preparation of the compounds of formulae (I), (Ia), (Ib), and (Ic).

The present invention covers intermediate compounds of formula (III): 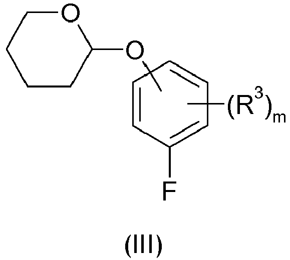 in which m and R³ are as defined for the compounds of general formula (I).

The present invention covers intermediate compounds of formula (V): 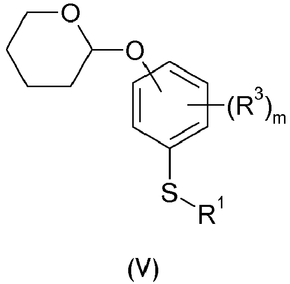 in which m, R¹ and R³ are as defined for the compounds of general formula (I), *supra.*

The present invention covers intermediate compounds of formula (VI): 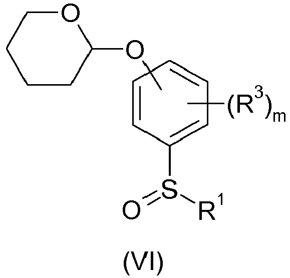 in which m, R¹ and R³ are as defined for the compounds of general formula (I), *supra.*

The present invention covers intermediate compounds of formula (VII): 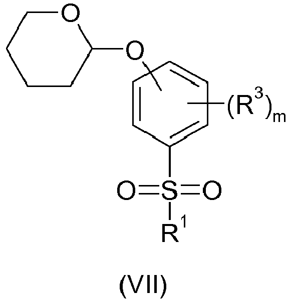 in which m, R¹ and R³ are as defined for the compounds of general formula (I), *supra.*

The present invention covers intermediate compounds of formula (VIII): 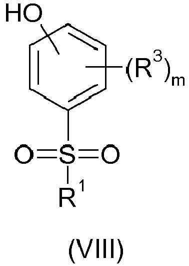 in which m, R¹ and R³ are as defined for the compounds of general formula (I), *supra.*

The present invention covers intermediate compounds of formula (X): 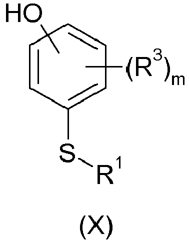 in which m, R¹ and R³ are as defined for the compounds of general formula (I), *supra.*

In particular, the present invention covers intermediate compounds which are disclosed in the Example Section of this text, infra.

It is apparent for one of ordinary skilled in the art, that the methods according to this invention can also be used for making derivatives of the compounds of formula (I).

### EXPERIMENTAL SECTION

The following Table lists the abbreviations used in this paragraph, and in the Examples Section. NMR peak forms are stated as they appear in the spectra, possible higher order effects have not been considered.

| **Abbreviation** | **Meaning** |
|---|---|
| Ac | acetyl |
| br | broad |
| BuPAd₂ | Di(1-adamantyl)-n-butylphosphine |
| D / d | doublet |
| Dd / dd | doublet of doublets |
| DMF | *N,N*-dimethylformamide |
| DMSO | dimethyl sulfoxide |
| ESI | electrospray ionisation |
| h / hrs | hour / hours |
| M / m | multiplet |
| m.p. | melting point in °C |
| MS | mass spectrometry |
| MW | molecular weight |
| NMR | nuclear magnetic resonance spectroscopy: chemical shifts (δ) are given in ppm. |
| q | quartet |
| Quin / quin | quintett |
| Rt / r. t. | room temperature |
| RT | retention time in minutes |
| s | singlet |
| sept | septet |
| t | triplet |
| UPLC | ultra performance liquid chromatography |

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae such as "hydrochloride", "trifluoroacetate", "sodium salt", or "x HCl", "x CF₃COOH", "x Na⁺", for example, are to be understood as not a stoichiometric specification, but solely as a salt form.

This applies analogously to cases in which synthesis intermediates or example compounds or salts thereof have been obtained, by the preparation and/or purification processes described, as solvates, such as hydrates with (if defined) unknown stoichiometric composition.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallisation. In some cases, impurities may be stirred out using a suitable solvent. In some cases, the compounds may be purified by chromatography, particularly flash chromatography, using for example pre-packed silica gel cartridges, *e.g.* from Separtis such as Isolute® Flash silica gel (silica gel chromatography) or Isolute® Flash NH2 silica gel (aminophase-silica-gel chromatography) in combination with a suitable chromatographic system such as a Flashmaster II (Separtis) or an Isolera system (Biotage) and eluents such as, for example, gradients of hexane/ethyl acetate or DCM/methanol. In some cases, the compounds may be purified by preparative HPLC using, for example, a Waters autopurifier equipped with a diode array detector and/or on-line electrospray ionisation mass spectrometer in combination with a suitable pre-packed reverse phase column and eluants such as, for example, gradients of water and acetonitrile which may contain additives such as trifluoroacetic acid, formic acid or aqueous ammonia.

### Examples

### 2-(2-bromo-5-fluorophenoxy)tetrahydro-2H-pyran

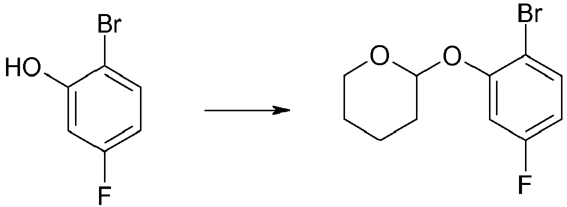

630.9g (7.5mol) Dihydropyran were dissolved in 630,9g dichloromethane and 62.8g (0.25mol) pyridinium-p-toluenesulfonate were added. The mixture was stirred at room temperature until complete dissolution. Then a mixture of 477.5g (2.5mol) 2-bromo-5-fluorophenol in 477.5g dichloromethane was dosed over 2.5h. The reaction was exothermic and up to 40°C internal temperature was reached. After addition the reaction mixture was stirred at room temperature overnight.

For work up the reaction mixture was extracted twice with 750ml 1N aqueous sodium hydroxide and once with 250ml saturated sodium chloride solution. The solvents were removed by distillation and the resulting viscous oil (756.7g, 111.3% of theory) was used in the next step without further purification.

### 2-[2-bromo-5-(methylsulfanyl)phenoxy]tetrahydro-2H-pyran

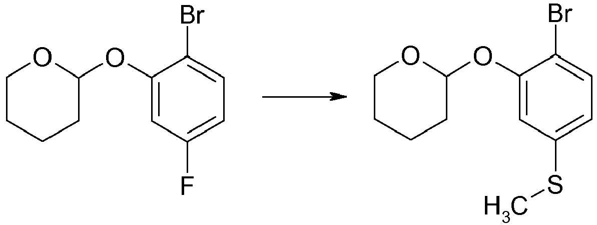

381.8g (1.25mol) of the crude product 2-(2-bromo-5-fluorophenoxy)tetrahydro-2H-pyran prepared as described above, 1125g DMF and 113.9g (1.625mol) sodium methylthiolate were charged into a reactor and heated under stirring to 60°C. Then the mixture was stirred at 60°C for 5-6h. The reaction mixture was concentrated by distillation (75°C, 40mbar) to an oily residue of 595g to which 850g isopropyl acetate was added.

The resulting mixture was extracted with 2000ml of water, then twice with 500ml water and once with 500ml of an 10% aqueous sodium chloride solution. The mixture was dried with a small amount of sodium sulfate and the solvents were removed by distillation. 375.6g (99.1% of theory) of the title product was obtained.

### 2-[2-bromo-5-(methylsulfonyl)phenoxy]tetrahydro-2H-pyran

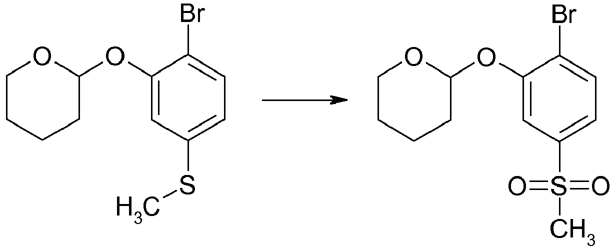

The complete crude product 2-[2-bromo-5-(methylsulfanyl)phenoxy]tetrahydro-2H-pyran prepared as described above (375.6g, "1.24mol") was dissolved in 1878g methanol and 2.04g (6.2mmol) sodium wolframate(VI) dihydrate and 24.8g (32mmol) 12.5% aqueous sulfuric acid were added. The mixture was stirred at 40-45°C and then 337.1g (3.47mol) 35% aqueous hydrogen peroxide solution was dosed over 20min below 55°C. After addition the mixture was stirred at 45-50°C for 4h and at room temperature overnight.

For work up 250 ml of a saturated aqueous solution of sodium thiosulfate was dosed until a KI/starch paper test was negative. The internal temperature was kept below 40°C. The methanol was distilled off and the distillation residue was extracted with 1750g ethyl acetate. The combined organic phase was filtered and the solvent was removed by distillation giving 347.2g (111.6% of theory) of the title product.

### 1-bromo-2-methoxy-4-(methylsulfonyl)benzene

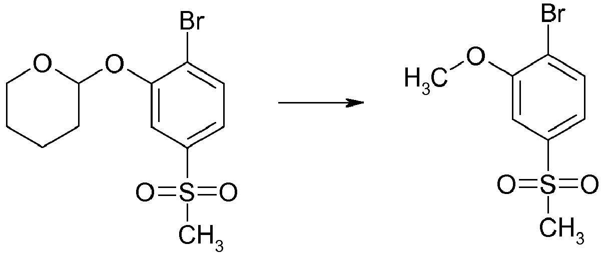

The complete crude product 2-[2-bromo-5-(methylsulfonyl)phenoxy]tetrahydro-2H-pyran prepared as described above (347.2g, "1.24mol") was dissolved in 2447g acetone. 256.9g (1.86mol) ground potassium carbonate was added and then 171.9g (1.36mol) dimethyl sulfate was dosed over 5min with stirring. The mixture was stirred at 55-60°C internal temperature for 2.5h. Then the mixture was cooled to room temperature, the inorganic precipitate was filtered and washed with 700g acetone. The combined filtrate was concentrated to 715g by distillation and 715g diisopropylether were slowly added. The resulting mixture was stirred over night at room temperature. The precipitate was filtered, washed twice with 125ml diisopropylether and dried under vacuum at 50°C.

An amount 222.7g (67.8% of theory over all 4 steps) was obtained.

The mother liquor was concentrated to 215g by distillation. The resulting precipitate was filtered and washed twice with 200ml diisopropylether and dried as above.

A second crop of 21.5g (6.5% of theory over all 4 steps) was obtained.

## Claims

1. Method for preparing a compound of general formula (I): 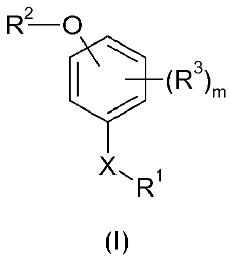 in which :
X represents S, S(=O) or S(=O)₂;
R¹ represents a group selected from: C₁-C₆-alkyl-, C₃-C₆-cycloalkyl-;
R² represents a group selected from: C₁-C₆-alkyl-, halo-C₁-C₆-alkyl-, C₃-C₆-cycloalkyl-;
R³ represents chloro, bromo or iodo, or a group selected from: C₁-C₆-alkyl-, C₃-C₆-cycloalkyl-; and
m represents an integer of 0, 1, 2, 3 or 4;
the method being **characterized in that** an intermediate compound of general formula (III): 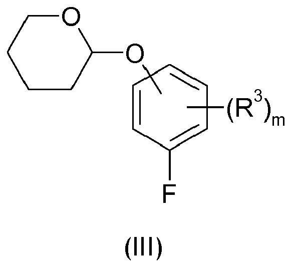 in which R³ is as defined for the compounds of general formula (I), *supra;*
is allowed to react with an alkanethiolat of general formula (IV):
R¹-S⁻X⁺ (IV)
in which R¹ is as defined for the compounds of general formula (I), *supra,* and X⁺ is a counter ion to the negatively charged sulphur ion, preferably Na⁺;
thus providing a compound of formula (V):

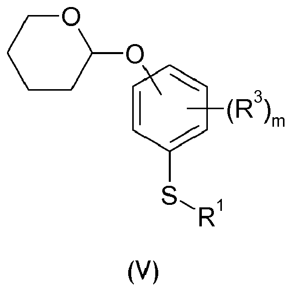

2. The method according to claim 1, wherein X represents S.

3. The method according to claim 1, wherein X represents S(=O).

4. The method according to claim 1, wherein X represents S(=O)₂.

5. The method according to any one claims 1 to 4, wherein the method comprises the step of converting the compound of formula (III): 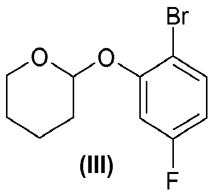 into the compound of formula (V):

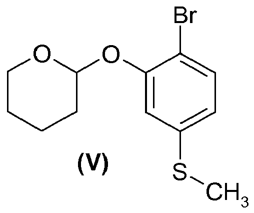

6. Intermediate compound of formula (III): 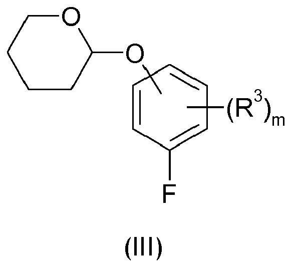 in which
R³ represents chloro, bromo or iodo, or a group selected from: C₁-C₆-alkyl-, C₃-C₆-cycloalkyl-; and
m represents an integer of 0, 1, 2, 3 or 4.

7. Intermediate compound of formula (V): 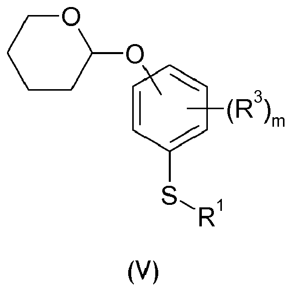 in which
R¹ represents a group selected from: C₁-C₆-alkyl-, C₃-C₆-cycloalkyl-;
R³ represents chloro, bromo or iodo, or a group selected from: C₁-C₆-alkyl-, C₃-C₆-cycloalkyl-; and
m represents an integer of 0, 1, 2, 3 or 4.

8. Intermediate compound of formula (VI): 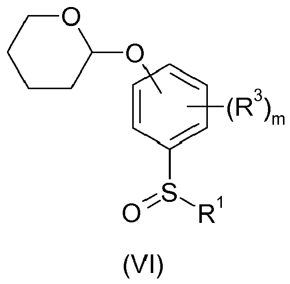 in which
R¹ represents a group selected from: C₁-C₆-alkyl-, C₃-C₆-cycloalkyl-;
R³ represents chloro, bromo or iodo, or a group selected from: C₁-C₆-alkyl-, C₃-C₆-cycloalkyl-; and
m represents an integer of 0, 1, 2, 3 or 4.

9. Intermediate compound of formula (VII): 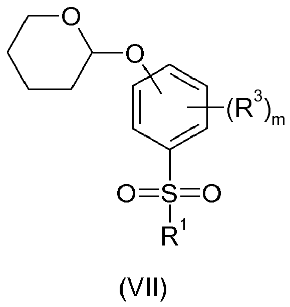 in which
R¹ represents a group selected from: C₁-C₆-alkyl-, C₃-C₆-cycloalkyl-;
R³ represents chloro, bromo or iodo, or a group selected from: C₁-C₆-alkyl-, C₃-C₆-cycloalkyl-; and
m represents an integer of 0, 1, 2, 3 or 4.

10. Intermediate compound of formula (VIII): 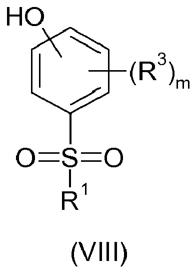 in which
R¹ represents a group selected from: C₁-C₆-alkyl-, C₃-C₆-cycloalkyl-;
R³ represents chloro, bromo or iodo, or a group selected from: C₁-C₆-alkyl-, C₃-C₆-cycloalkyl-; and
m represents an integer of 0, 1, 2, 3 or 4.

11. Intermediate compound of formula (X): 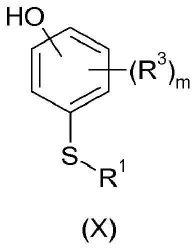 in which
R¹ represents a group selected from: C₁-C₆-alkyl-, C₃-C₆-cycloalkyl-;
R³ represents chloro, bromo or iodo, or a group selected from: C₁-C₆-alkyl-, C₃-C₆-cycloalkyl-; and
m represents an integer of 0, 1, 2, 3 or 4.

12. Intermediate compound selected from:
2-((2-bromo-5-fluorophenoxy)tetrahydro-2H-pyran,
2-[2-bromo-5-(methylsulfanyl)phenoxy]tetrahydro-2H-pyran,
2-[2-bromo-5-(methylsulfinyl)phenoxy]tetrahydro-2H-pyran,
2-[2-bromo-5-(methylsulfonyl)phenoxy]tetrahydro-2H-pyran,
2-bromo-5-(methylsulfonyl)phenol.

13. Use of an intermediate compound of any one of claims 6, 7, 8, 9, 10, 11 and 12 for the preparation of a compound of formula (I) as defined in claim 1.
